# EUROPEAN PATENT SPECIFICATION

(11) **EP 1 850 768 B1**
(45) Date of publication and mention of the grant of the patent: **18.04.2012**
(21) Application number: 03788001.0
(22) Date of filing: 05.08.2003
(51) Int. Cl.: A61B 17/34

(54) **CURVED TROCAR FOR THORACOSCOPY**
GEBOGENER TROKAR FÜR THORAKOSKOPIE
TROCART COURBE POUR THORACOSCOPIE

(30) Priority: 19.08.2002 IT PV20020003 U
(43) Date of publication of application: 07.11.2007
(73) Proprietor: Rizzo, Sebastiano, 27100 Pavia (IT)
(72) Inventor: Rizzo, Sebastiano, 27100 Pavia (IT)
(74) Representative: Pizzoli, Antonio
(86) International application number: PCT/IT2003/000501
(87) International publication number: WO 2004/016184

(56) References cited:
- US-A- 1 906 678
- US-A- 4 428 379
- US-A- 4 863 430
- US-A- 4 877 037

## Description

DESCRIPTION: the curved trocar for thoracoscopy consists of a rigid cannula wherein a short arm continues through a 60°-70° angle curve into a long arm provided with a support and blocking system; an internal stylet is formed by a body, a conic tip and a handle, in nylon or other resistant material.

TECHNICAL FIELD: medical field; surgical device (support for thoracoscopy and thoracocentesis).

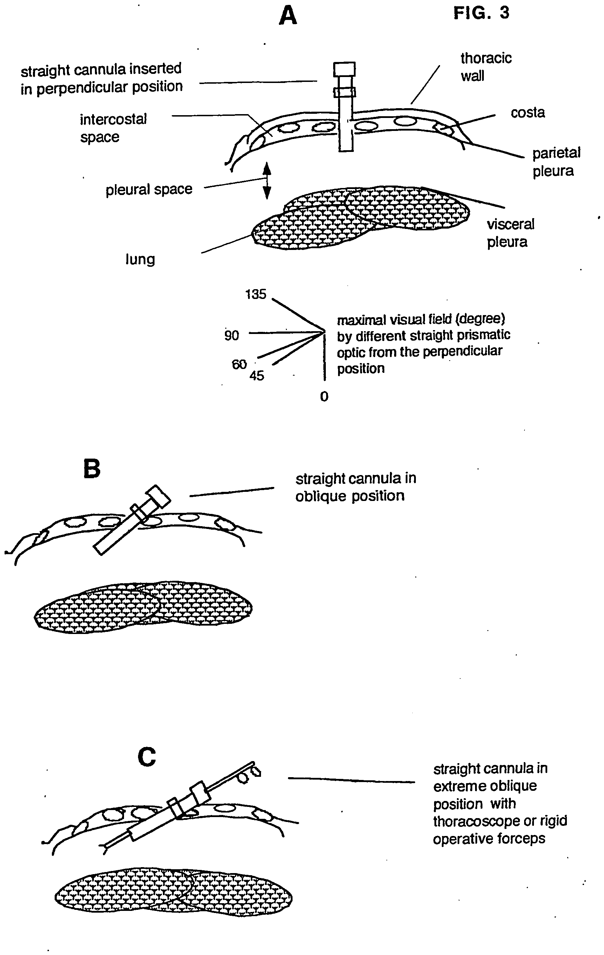

BACKGROUND ART: straight trocars for introducing rigid or flexible thoracoscopes are still in use in medical field since 1910 (1). Such devices consist of a straight cannula mounted on a straight stylet. The thoracic wall is pierced by a mounted trocar. The stylet is removed and the cannula is left as a temporary, artificial path to introduce the thoracoscope. The cannula, inserted perpendicularly through the intercostal space, sustains the thoracoscope, as illustrated in figs. 3A, 3B, 3C. While maintaining perpendicularly the system cannula-thoracoscope, the internal vision may vary from 0° to 135°, depending on the direct or lateral prism used. To overcome such limit, it is necessary to incline the cannula from the perpendicular position to a very oblique one, as illustrated in figs. 3-B, 3C. This movement is necessary to observe and treat structures or lesions far from the point where the cannula is inserted. The movement in the intercostal space may be carried on in two manners: 1) along its major or, 2) minor axis. In the former case the pressure is carried on the intercostal muscles by inclining the cannula with backward-frontward and anterior-posterior movements; in the latter case, the pressure is carried on the ribs by cranio-caudal movements. In both cases, especially in the second one, a strong, painful pressure is carried on the patient. The observation of the thoracic cavity may result limited. Such a limit may manifest itself when particular treatments are needed (scraping, biopsy, resection, etc.). A wider visual field may be obtained by using a flexible fiberoptic bronchoscopes (fiberbronchoscope) (2); but, even if the visual-operative system is flexible, the straight cannula maintains many of the mentioned limitations. A recent thoracofiberscope (Thoracofiberscope LFT, Olympus Optical Co Ltd; Tokio, Japan) (3) is simply a modified fiberbronchoscope. Using a straight cannula as a support, it partially maintains the same limits.
(1) Jacobaeus, 1910.
(2) Seno, 1974.
(3) Maclean, 1998.

US 4428379 discloses a biopsy needle which is straight and which carries therewithin a stylette.

US 4863430 discloses a thin and flexible trocar which can be inserted in an elongated arcuate tubular body of a cannula. Due to its particular arcuate shape, this cannula is not suitable for thoracoscopy, in particular for observing areas of the pleural space which are not close to the insertion point of the cannula.

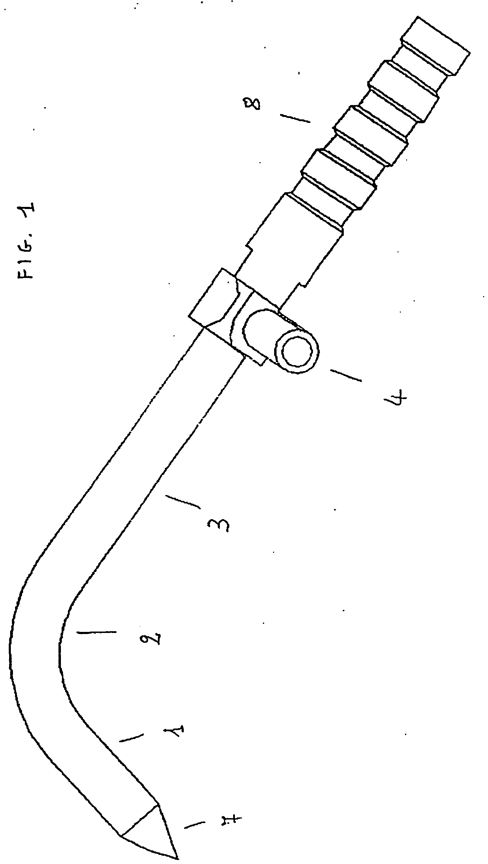

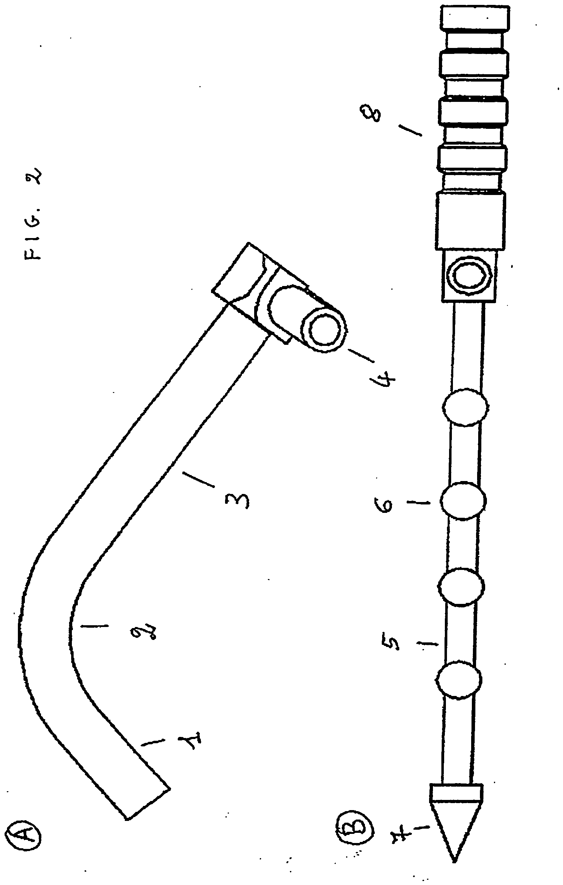

DISCLOSURE OF INVENTION : the curved trocar for thoracoscopy consists of an external rigid cannula in which a short arm (1) continues through a 60° - 70° angle curve (2) into a long arm (3) provided with a support and blocking system (4), as shown in figs. 1 and 2A; an internal stylet is formed by a÷ body (5) with blocking spheres (6), a conic tip and a handle (7, 8), in nylon or other resistant material. Once the trocar short arm is inserted into the pleural space through the intercostals space, the stylet is removed and the fiberbronchoscope is introduced through the cannula. The system is shown in figs. 1 and 2B. By cranio-caudal and backward-frontward movements, the inclination, vision and operability are more accentuated and multidirectional.

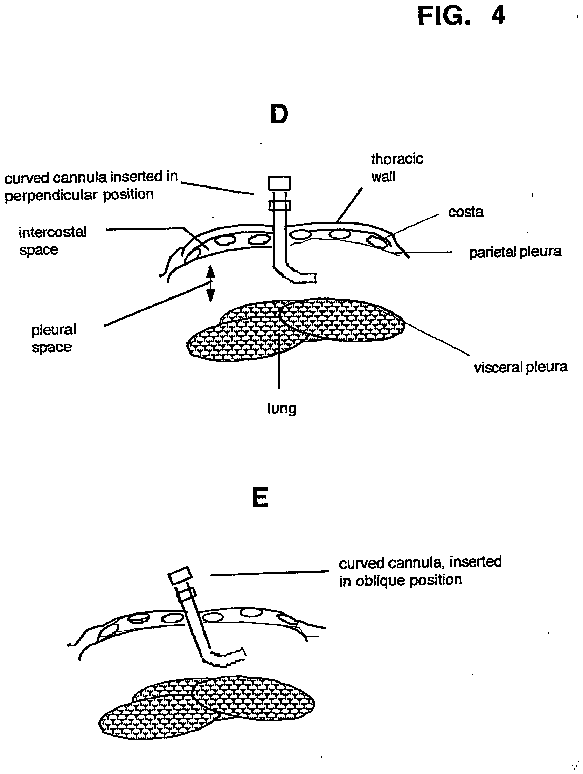

The cannula-fiberbronchoscope system, shown in figs. 4-D, E works mainly on the own longitudinal axis with up-down and rotation movements. The lateral inclination is minimal. The curved cannula together with the flexibility of the optic fibers increases the possibility to inspect and operate in the remotest parts of the pleural cavity. That reduces difficulties and discomfort to the patients who underwent the examination under local anesthesia.

BRIEF DESCRIPTION OF DRAWINGS: Fig. 1: a view of the mounted trocar.

Fig. 2: a view of the unmounted trocar. Fig. 3A: a view of the classic straight cannula inserted in the pleural space through the intercostal space, in perpendicular position for a direct vision. Fig. 3B: a view of the classic straight cannula inserted and held in oblique position. Fig. 3C: a view of the classic straight cannula, inserted and held in extreme oblique position, guiding the straight thoracoscope. Fig. 4D: curved cannula inserted and held in perpendicular position; as in the drawing, the cannula may be rotated in any direction; pulling it up or introducing it the direct vision is possible. Fig. 4-E: curved cannula inserted and held in oblique position; by introducing and flexing the tip of a fiberbronchoscope, a retrograde vision and operabitity are possible.

BEST MODE FOR CARRYING OUT THE INVENTION: the curved trocar may be produced in steel, nylon or plastic material in the respect of the rules for the applicability to medical field. Plastic material should be sufficiently resistant to pierce the chest wall and not be deformed by the intercostal muscles or by the procedures.

## Claims

1. Curved trocar for thoracoscopy composed by an external rigid cannula and by an internal stylet suitable for being removed from this cannula, **characterized in that** the cannula has a short arm (1) which continues through a curve **(2)** into a long arm **(3)** provided with a handle-blocking system **(4)**, and **in that** the internal stylet is formed by a body **(5)** with blocking spheres **(6)** anchored to it, a conic point **(7)** and a handle **(8)** being arranged respectively at the head and tail of said body **(5),** wherein said spheres **(6)** are suitable for maintaining the body **(5)** of the stylet firm toward the internal wall of said cannula.

2. A curved trocar for thoracoscopy AS CLAIMED IN CLAIM 1 **characterized in that** said curve **(2)** is comprised between 60° and-70°.

3. A curved trocar for thoracoscopy AS CLAIMED IN CLAIM 1 **characterized in that** said body **(5)** is made of a flexible and resistant material to overcome the curve **(2)** of said cannula.

4. A curved trocar for thoracoscopy AS CLAIMED IN CLAIM 3 **characterized in that** said body **(5)** is made of nylon.

5. A curved trocar for thoracoscopy AS CLAIMED IN CLAIM 1 **characterized in that** said spheres **(6)** are made of nylon.

## Patentansprüche

1. Gekrümmter Trokar für eine Thorakoskopie, bestehend aus einer äußeren starren Kanüle und einem inneren Stylet, das geeignet ist, aus dieser Kanüle entfernt zu werden, **dadurch gekennzeichnet, dass** die Kanüle einen kurzen Arm (1) aufweist, der sich über eine Krümmung (2) in einem langen Arm (3) fortsetzt, der mit einem Griffblockierungssystem (4) versehen ist, und dadurch, dass das innere Stylet aus einem Körper (5) mit an diesem verankerten Blockierkugeln, einer kegelförmigen Spitze (7) und einem Griff (8) besteht, die an dem Vorder- bzw. dem Endstück des Körpers (5) angeordnet sind, wobei die Kugeln (6) geeignet sind, den Körper (5) des Stylets an der Innenwand der Kanüle abzustützen.

2. Gekrümmter Trokar für eine Thorakoskopie nach Anspruch 1, **dadurch gekennzeichnet, dass** die Krümmung (2) zwischen 60° und 70° beträgt.

3. Gekrümmter Trokar für eine Thorakoskopie nach Anspruch 1, **dadurch gekennzeichnet, dass** der Körper (5) aus einem flexiblen und widerstandsfähigen Material besteht, so dass er die Krümmung (2) der Kanüle überwinden kann.

4. Gekrümmter Trokar für eine Thorakoskopie nach Anspruch 3, **dadurch gekennzeichnet, dass** der Körper (5) aus Nylon besteht.

5. Gekrümmter Trokar für eine Thorakoskopie nach Anspruch 1, **dadurch gekennzeichnet, dass** die Kugeln (6) aus Nylon bestehen.

## Revendications

1. Trocart courbe pour thoracoscopie constitué d'une canule rigide externe et d'un stylet interne convenant pour être retiré de ladite canule, **caractérisé en ce que** la canule possède un bras court (1) qui se poursuit par une courbe (2) en un bras long (3) munie d'un système de blocage de poignée (4), et le stylet interne est formé d'un corps (5) avec des sphères de blocage (6) ancrées dessus, une pointe conique (7) et une poignée (8) étant agencées respectivement au niveau de l'avant et de l'arrière dudit corps (5), dans lequel lesdites sphères (6) conviennent pour maintenir le corps (5) du stylet fermement par rapport à la paroi interne de ladite canule.

2. Trocart courbe pour thoracoscopie selon la revendication 1, **caractérisé en ce que** ladite courbe (2) est comprise entre 60° et 70°.

3. Trocart courbe pour thoracoscopie selon la revendication 1, **caractérisé en ce que** ledit corps (5) est réalisé en un matériau flexible et résistant pour suivre la courbe (2) de ladite canule.

4. Trocart courbe pour thoracoscopie selon la revendication 3, **caractérisé en ce que** ledit corps (5) est constitué de nylon.

5. Trocart courbe pour thoracoscopie selon la revendication 1, **caractérisé en ce que** lesdites sphères (6) sont constituées de nylon.
